# FASCICULE DE BREVET EUROPEEN

(11) **EP 0 913 135 B1**
(45) Date de publication et mention de la délivrance du brevet: **01.10.2003**
(21) Numéro de dépôt: 98420184.8
(22) Date de dépôt: 13.10.1998
(51) Int. Cl.: A61F 2/38

(54) **Pièce fémorale pour une prothèse de genou**
Femorales Komponent für eine Knieprothese
Femoral component for a knee prothesis

(30) Priorité: 14.10.1997 FR 9713072
(43) Date de publication de la demande: 06.05.1999
(73) Titulaire: Tornier SA, 38330 Saint Ismier (FR)
(72) Inventeur: Timoteo, Michel, 38410 Saint Martin D'Uriage (FR)
(74) Mandataire: Schmitt, John

(56) Documents cités:
- FR-A- 2 712 799
- US-A- 5 116 375
- US-A- 5 405 398
- US-A- 5 549 687

## Description

L'invention se rapporte à une pièce fémorale pour prothèse de genou.

La pièce fémorale d'une prothèse de genou est constituée de façon connue, de deux condyles latéraux, à savoir un condyle externe et un condyle interne, lesquels définissent un espace inter-condylien. Les condyles latéraux sont destinés à coopérer avec un insert rapporté sur un plateau tibial, lui-même ancré au niveau de l'extrémité supérieure du tibia.

Ce type de pièce fémorale est utilisé lorsqu'on met en place une prothèse de genou chez un patient, dont les ligaments, et notamment le ligament croisé postérieur, sont dans un état correct, c'est à dire lorsqu'ils sont susceptibles de maintenir ladite pièce fémorale sur l'ensemble insert/plateau tibial, en évitant ainsi tout mouvement de tiroir.

Lorsque le ligament croisé postérieur n'est plus à même de remplir sa fonction, on a recourt, pour le remplacer, à utiliser une pièce fémorale dont l'espace inter-condylien est en partie obturé par un troisième condyle, lequel permet la postéro-stabilisation de la prothèse, lors de la flexion du tibia sur le fémur.

Au vu de ces deux types de cas de figure, il est apparu intéressant de proposer une pièce fémorale qui puisse être au choix utilisée en présence ou en l'absence du ligament croisé postérieur.

Ce type de pièce fémorale a notamment été décrit dans le document FR-A-2 710 258, dans lequel il est proposé d'adapter dans l'espace intercondylien, une pièce support escamotable, susceptible de recevoir un troisième condyle.

En pratique, lorsque qu'il n'est pas nécessaire de remplacer le ligament croisé postérieur, le chirurgien implante définitivement la pièce fémorale sans y fixer le troisième condyle.

En revanche, en l'absence de ligament croisé postérieur, le chirurgien met en place le troisième condyle avant implantation de la pièce fémorale.

Ce type de pièce fémorale ne permet malheureusement pas, après mise en place de la prothèse sur un genou possédant encore son ligament croisé postérieur, d'adapter, en cas de rupture ultérieure de celui-ci, un troisième condyle, sans retrait total de la pièce fémorale du fémur, lequel engendre un traumatisme important.

On connaît également d'après le document US 5 405 398 qui décrit une pièce fémorale pour prothèse de genou destinée à coopérer avec un insert rapporté sur un plateau tibial ancré au niveau de l'extrémité supérieure du tibia.

Le document antérieur décrit que la pièce fémorale est ancrée au niveau de l'extrémité inférieure du fémur, et présente deux condyles latéraux coopérant avec ledit insert et définissant un espace inter-condylien, chacun des deux condyles latéraux présente des agencements aptes à assurer la mise en place ou le retrait d'un élément dans l'espace inter-condylien.

Le document antérieur montre des agencements qui sont constitués :
- d'au moins un orifice traversant qui est ménagé sur les faces latérales de chacun des deux condyles latéraux, lesdits orifices étant alignés ;
- et d'au moins une vis dont l'extrémité présente un filetage destiné à coopérer avec un filetage ménagé au niveau de l'orifice de l'un des deux condyles latéraux.

Le but de l'invention est donc de proposer une pièce fémorale pour prothèse de genou sur laquelle on puisse adapter, une fois ladite pièce fémorale implantée, un troisième condyle et ce, sans avoir à retirer la pièce fémorale du fémur.

Pour résoudre ce problème, l'invention propose une pièce fémorale pour prothèse de genou destinée à coopérer avec un insert rapporté sur un plateau tibial ancré au niveau de l'extrémité supérieure du tibia, ladite pièce fémorale étant elle-même ancrée au niveau de l'extrémité inférieure du fémur et présentant deux condyles latéraux coopérant avec ledit insert et définissant un espace inter-condylien susceptible de recevoir un troisième condyte amovible.

La pièce fémorale se caractérise en ce que chacun des deux condyles latéraux présente des agencements aptes à assurer la mise en place ou le retrait d'un troisième condyle, après ancrage définitif de la pièce fémorale sur le fémur.

En d'autres termes, l'invention permet d'adapter un troisième condyle sur une pièce fémorale définitivement ancrée dans le fémur, lorsqu'il devient nécessaire de supprimer le ligament postérieur croisé.

Selon une première caractéristique de l'invention, lesdits agencements sont constitués d'au moins un orifice traversant, ménagé sur les faces latérales de chacun des deux condyles latéraux, lesdits orifices étant destinés à recevoir un moyen de solidarisation du troisième condyle aux deux condyles latéraux.

Avantageusement, les orifices sont au nombre de deux et sont ménagés selon un axe vertical.

Pour permettre l'adaptation d'un troisième condyle au niveau des deux condyles latéraux, ledit troisième condyle présente des agencements complémentaires destinés à coopérer avec les agencements des condyles latéraux.

En pratique, les agencements complémentaires sont constitués par au moins un orifice traversant ménagé sur les faces latérales du troisième condyle.

Avantageusement, les orifices sont au nombre de deux par face et sont alignés avec les orifices prévus sur les faces latérales des condyles latéraux.

Afin d'obtenir la postéro-stabilisation de la prothèse du genou lors de la flexion du tibia sur le fémur, le troisième condyle présente une forme semi-cylindrique d'épaisseur inférieure à celle de l'espace inter-condylien, chacune des faces latérales du troisième condyle étant munie d'une protubérance destinée à venir au contact des faces latérales internes des condyles latéraux, lesdites protubérances présentant deux orifices latéraux destinés à être positionnés en alignement avec les orifices des condyles latéraux.

Pour permettre la fixation amovible du troisième condyle aux condyles latéraux, le moyen de solidarisation est constitué d'au moins une vis dont l'une des extrémités présente un filetage destiné à coopérer avec un filetage ménagé dans un orifice de l'un des deux condyles latéraux.

Il suffit donc, lors de la mise en place du troisième condyle de positionner chacun des orifices en regard l'un de l'autre, puis d'introduire la vis à travers les orifices puis enfin visser afin de permettre la fixation du troisième condyle sur la pièce fémorale.

L'invention et les avantages qui en découlent ressortiront mieux de l'exemple de réalisation suivant à l'appui des figures annexées.

La figure 1 est une vue en perspective de la pièce fémorale de l'invention.

La figure 2 est une vue dans le sens antéro-postérieur de la pièce fémorale selon la figure 1.

La figure 3 est une vue dans le sens postéro-antérieur selon la figure 1.

La figure 4 est une vue de dessus de la pièce fémorale selon la figure 1.

Les figures 5 et 6 sont des représentations schématiques, respectivement de profil et de face du troisième condyle conforme à l'invention.

On désigne par la référence générale (1 ) la pièce fémorale de l'invention. Celle-ci comporte de façon connue deux condyles latéraux (2, 3), définissant un espace inter-condylien (4).

Cette pièce fémorale est destinée à être ancrée au niveau de l'extrémité inférieure du fémur, maïs également à coopérer avec un insert rapporté sur un plateau tibial ancré au niveau de l'extrémité supérieure du tibia. L'insert et le tibia n'ont pas été représentés dans la mesure où ils ne font pas directement l'objet de l'invention.

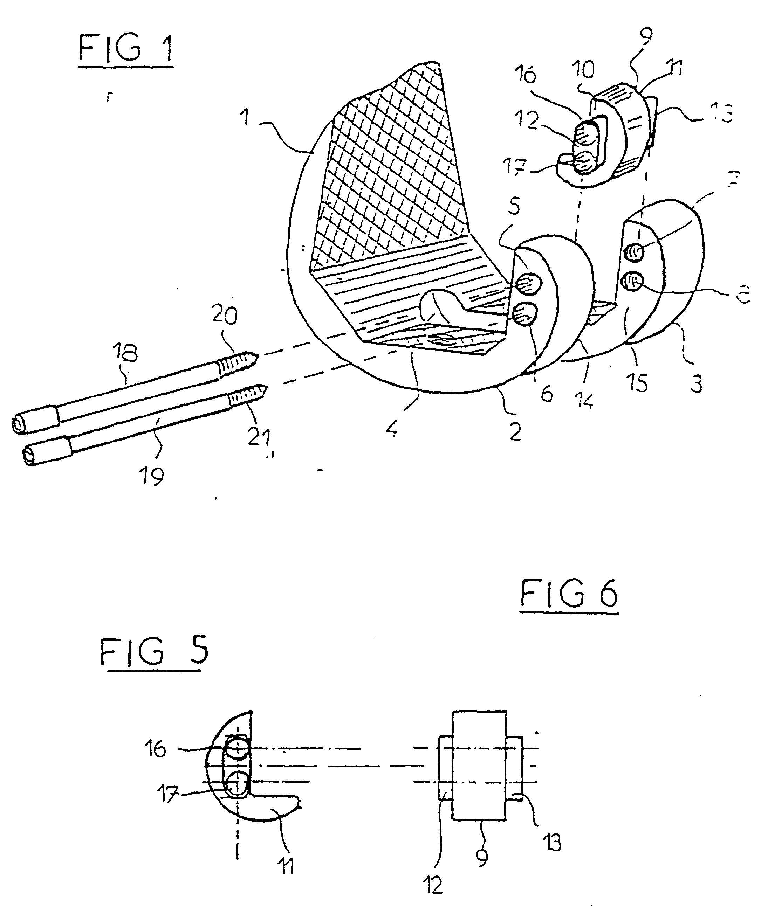

Comme le montre la figure 1, chacun des deux condyles latéraux (2, 3) présente sur leur face latérale, deux orifices traversant (5, 6, 7 8) ménagés selon un axe vertical.

Lorsque le ligament croisé postérieur du patient est conservé, la pièce fémorale peut être implantée telle quelle au niveau de l'extrémité inférieure du fémur, c'est à dire en l'absence d'un troisième condyle.

En revanche, si le ligament croisé postérieur est absent, il est nécessaire d'implanter une prothèse postéro-stabilisée.

Pour ce faire, on a recourt à la mise en place d'un troisième condyle (9) dans l'espace inter-condylien (4), au niveau des extrémités postérieures libres des condyles latéraux (2, 3).

Comme déjà dit, le troisième condyle peut être mis en place une fois la pièce fémorale implantée, sans nécessiter le retrait de ladite pièce.

Selon un mode préféré de réalisation de l'invention, ce troisième condyle se présente sous forme semi-cylindrique, dont la largeur l est inférieure à la largeur L de l'espace inter-condylien.

Avantageusement, le troisième condyle (9) présente sur chacune de ses faces latérales (10, 11) une protubérance (12, 13) destinée à venir en contact avec les faces latérales (14, 15) internes des condyles latéraux. Chaque protubérance (12, 13) présente en outre deux orifices (16, 17), lesquels sont réalisés de telle sorte à, une fois le condyle (9) en place, se trouver en alignement avec les orifices (5 à 8) des condyles latéraux (2, 3).

Lorsque le troisième condyle est mis en place, c'est à dire lorsque tous les orifices sont en alignement deux à deux, on introduit à travers lesdits orifices un moyen de solidarisation du troisième condyle aux condyles latéraux.

En pratique, ce moyen de solidarisation se présente sous forme d'une ou plusieurs vis (18, 19), dont l'extrémité (20) est filetée et destinée à coopérer avec un filetage correspondant ménagé dans les orifices de l'un des deux condyles latéraux.

Ainsi, lorsqu'on désire mettre en place le troisième condyle, il suffit d'introduire les vis (18, 19) dans les orifices correspondants respectivement (5, 16) et (6, 17), puis de bloquer la vis dans l'orifice fileté (7).

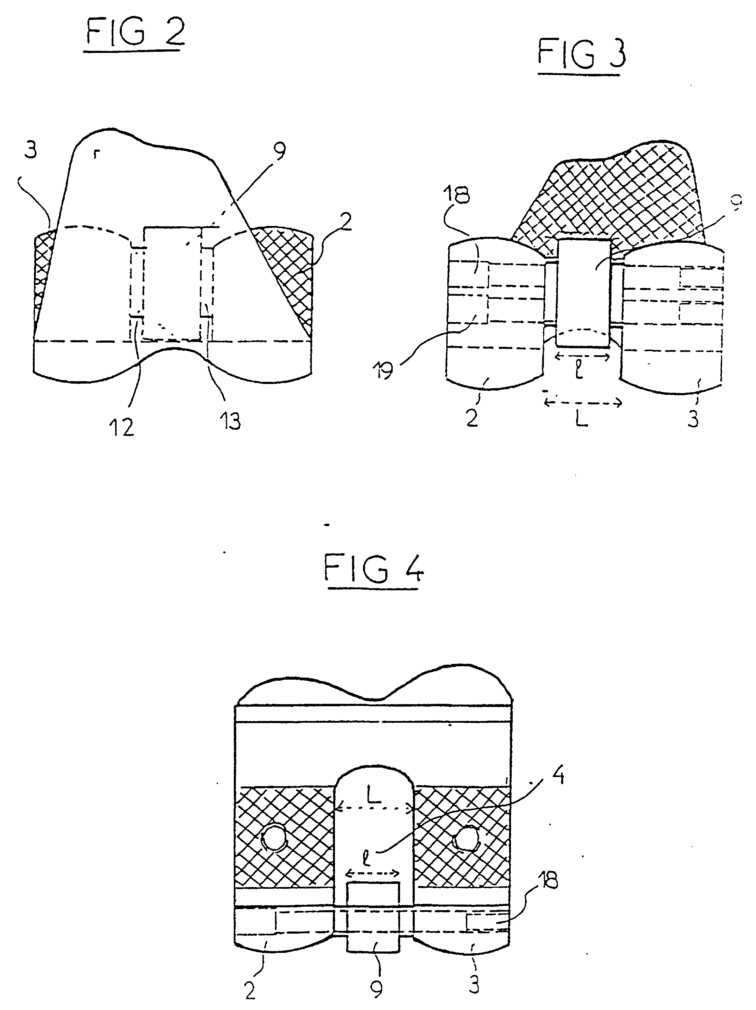

La figure 2 montre la pièce fémorale de l'invention sur laquelle est monté un troisième condyle.

Sur la figure 3, on a représenté le passage des vis (18 et 19) respectivement dans les orifices (5, 7, 16) et (6, 8, 17).

Comme déjà dit, lorsque le genou du patient est muni du ligament croisé postérieur, le chirurgien implante la pièce fémorale de l'invention comportant deux condyles.

Si par la suite, le ligament croisé postérieur vient à rompre ou ne remplit plus sa fonction, alors il est possible pour le chirurgien d'adapter le troisième condyle sur la prothèse implantée sans avoir à ôter celle-ci de l'os du fémur, ce qui permet d'éviter tout traumatisme de l'os.

L'invention telle que décrite présente donc un grand nombre d'avantages et permet notamment de transformer une prothèse de genou non postéro-stabilisée en prothèse postéro-stabilisée grâce à l'adjonction en per ou en post opératoire d'un troisième condyle, et ce sans qu'il ne soit nécessaire de retirer la pièce fémorale du fémur.

## Revendications

1. Pièce fémorale (1) pour prothèse de genou destinée à coopérer avec un insert rapporté sur un plateau tibial ancré au niveau de l'extrémité supérieure du tibia, ladite pièce fémorale étant elle-même ancrée au niveau de l'extrémité inférieure du fémur, et présentant deux condyles latéraux (2, 3) coopérant avec ledit insert et définissant un espace inter-condylien (4) susceptible de recevoir un troisième condyle (9) amovible, **caractérisée en ce que** chacun des deux condyles latéraux (2, 3) présente des agencements aptes à assurer la mise en place ou le retrait d'un troisième condyle (9), après ancrage définitif de la pièce fémorale sur le fémur, le troisième condyle (9) présentant une forme semi-cylindrique d'épaisseur l inférieure à celle L de l'espace inter-condylien (4), chacune des faces latérales (10, 11) du troisième condyle (9) étant munie d'une protubérance (12, 13) destinée à venir au contact des faces latérales intemes (14, 15) des condyles latéraux (2, 3), lesdites protubérances (13, 14) présentant deux orifices latéraux (16, 17) destinés à être positionnés en alignement avec des orifices (5, 6, 7, 8) des condyles latéraux (2, 3) pour recevoir un moyen de solidarisation du troisième condyle (9).

2. Pièce fémorale (1) pour prothèse de genou selon la revendication 2, **caractérisée en ce que** le moyen de solidarisation du troisième condyle aux condyles latéraux est constitué d'au moins une vis (18, 19) dont l'extrémité (20, 21) présente un filetage destiné à coopérer avec un filetage ménagé au niveau de l'orifice (7, 8) de l'un des deux condyles latéraux (2, 3).

3. Pièce fémorale (1) pour prothèse de genou selon la revendication 2, **caractérisée en ce que** les orifices (5, 6, 7, 8) sont au nombre de deux par face (5, 6, 7, 8) et sont ménagés selon un axe vertical.

## Patentansprüche

1. Oberschenkelknochenteil (1) für Knieprothese, dazu bestimmt, mit einem auf der Schienbeinplatte, die auf der Ebene des oberen Endes des Schienbeins verankert ist, angebauten Einsatz zusammenzuarbeiten, wobei der Oberschenkelknochenteil selbst auf der Ebene des unteren Endes des Oberschenkelknochens verankert ist und zwei seitliche Kondylen (2, 3) aufweist, die mit dem Einsatz zusammenarbeiten und einen Raum (4) zwischen Kondylen festlegen, der eine dritte abnehmbare Kondyle (9) aufnehmen kann, **dadurch gekennzeichnet, dass** jede der zwei seitlichen Kondylen (2, 3) Einrichtungen aufweist, die dazu geeignet sind, nach dem endgültigen Verankern des Oberschenkelknochenteils auf dem Oberschenkelknochen das Anbringen oder das Entfernen der dritten Kondyle zu gewährleisten, wobei die dritte Kondyle (9) eine halbzylindrischen Form mit einer Stärke l kleiner als die Stärke L des Raums (4) zwischen Kondylen aufweist, wobei jede der seitlichen Flächen (10, 11) der dritten Kondyle (9) mit einem Vorsprung (12, 13) versehen ist, der dazu bestimmt ist, mit den seitlichen internen Flächen (14, 15) der seitlichen Kondylen (2, 3) in Kontakt zu kommen, wobei die Vorsprünge (13, 14) seitliche Öffnungen (16, 17) aufweisen, die dazu bestimmt sind, mit den Öffnungen (5, 6, 7, 8) der seitlichen Kondylen (2, 3) gefluchtet zu werden, um ein Mittel zum festen Verbinden der dritten Kondyle (9) aufzunehmen.

2. Oberschenkelknochenteil (1) für Knieprothese nach Anspruch 1, **dadurch gekennzeichnet, dass** das Mittel zum festen Verbinden der dritten Kondyle mit den seitlichen Kondylen aus mindestens einer Schraube (18, 19) besteht, deren Ende (20, 21) ein Gewinde aufweist, das dazu bestimmt ist, mit einem Gewinde zusammenzuarbeiten, das auf der Ebene der Öffnung (7, 8) einer der zwei seitlichen Kondylen (2, 3) eingerichtet ist.

3. Oberschenkelknochenteil (1) für Knieprothese nach Anspruch 2, **dadurch gekennzeichnet, dass** es von den Öffnungen (5, 6, 7, 8) jeweils zwei pro Seite (5, 6, 7, 8) gibt und sie entlang einer senkrechten Achse angeordnet sind.

## Claims

1. Femoral component (1) for knee prosthesis intended to cooperate with an insert attached to a tibial plateau which is anchored at the upper end of the tibia, the said femoral component itself being anchored at the lower end of the femur and having two lateral condyles (2, 3) which cooperate with the said insert and define an intercondylar space (4) which is able to receive a third, removable condyle (9), **characterized in that** each of the two lateral condyles (2, 3) has arrangements permitting the fitting or removal of a third condyle (9) following definitive anchoring of the femoral component on the femur, the third condyle (9) having a semicylindrical shape with a thickness l smaller than that L of the intercondylar space (4), each of the lateral faces (10, 11) of the third condyle (9) being equipped with a protuberance (12, 13) intended to make contact with the inner lateral faces (14, 15) of the lateral condyles (2, 3), the said protuberances (12, 13) having two lateral orifices (16, 17) which are intended to be positioned in alignment with orifices (5, 6, 7, 8) of the lateral condyles (2, 3) in order to receive a means for securing the third condyle (9).

2. Femoral component (1) for knee prosthesis according to Claim 1, **characterized in that** the means for securing the third condyle to the lateral condyles consists of at least one screw (18, 19), the end (20, 21) of which has a thread intended to cooperate with a thread formed in the orifice (7, 8) of one of the two lateral condyles (2, 3).

3. Femoral component (1) for knee prosthesis according to Claim 2, **characterized in that** the orifices (5, 6, 7, 8) are arranged two orifices per face (5, 6, 7, 8) and they are formed along a vertical axis.
